(19) Europäisches Patentamt
European Patent Office
Office européen des brevets

(11) Veröffentlichungsnummer: **0 216 279**
**B1**

# (12) EUROPÄISCHE PATENTSCHRIFT

(45) Veröffentlichungstag der Patentschrift: 24.05.89

(51) Int. Cl.⁴: C 07 D 333/38

(21) Anmeldenummer: 86112679.5

(22) Anmeldetag: 13.09.86

**ERRATUM**

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE, COLONNE, LIGNE) | | | LAUTET BERICHTIGT:<br>SHOULD READ :<br>DEVRAIT ETRE LU : |
|---|---|---|---|---|
| in Gegenwart von Mn,Crȯ, Cr | 3 | 4 | 53 | in Gegenwart von Mn, Co, Cr |

Tag der Entscheidung über die Berichtigung
Date of decision on rectification: 12.07.89

Ausgabe- und Veröffentlichungstag:
Issue and publication date:
Date d'edition et de: 30.08.89

Patbl.Nr.
EPB no: 89/35

(19) Europäisches Patentamt
European Patent Office
Office européen des brevets

(11) Veröffentlichungsnummer: **0 216 279**
**B1**


(12) # EUROPÄISCHE PATENTSCHRIFT


(45) Veröffentlichungstag der Patentschrift: **24.05.89**

(51) Int. Cl.⁴: **C 07 D 333/38**

(21) Anmeldenummer: **86112679.5**

(22) Anmeldetag: **13.09.86**


(54) **Verfahren zur Herstellung von Halogenthiophen-2-carbonsäuren.**


(30) Priorität: **26.09.85 DE 3534286**

(43) Veröffentlichungstag der Anmeldung:
**01.04.87 Patentblatt 87/14**

(45) Bekanntmachung des Hinweises auf die Patenterteilung:
**24.05.89 Patentblatt 89/21**

(84) Benannte Vertragsstaaten:
**BE CH DE FR GB IT LI NL**

(56) Entgegenhaltungen:
**US-A- 2 462 697**
**US-A- 2 492 645**
**US-A- 4 230 873**



(73) Patentinhaber: **HOECHST AKTIENGESELLSCHAFT, Postfach 80 03 20, D-6230 Frankfurt am Main 80 (DE)**

(72) Erfinder: **Meldert, Helmut, Dr., Griesheimer Stadtweg 11, D-6230 Frankfurt am Main 80 (DE)**
Erfinder: **Mack, Karl-Ernst, Dr., Klingenbachstrasse 43, D-6200 Wiesbaden (DE)**
Erfinder: **Litterer, Heinz, Dr., Hardtstrasse 77, D-6208 Bad Schwalbach (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



EP 0 216 279 B1

ACTORUM AG

**Beschreibung**

Die Erfindung betrifft ein Verfahren zur Herstellung von Halogenthiophen-2-carbonsäuren der allgemeinen Formel

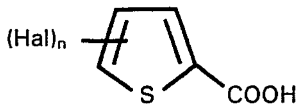

mit n = 1, 2
Hal = Halogen.

Halogenthiophen-2-carbonsäuren sind als wertvolle Zwischenprodukte für die Herstellung pharmazeutischer Präparate bekannt. Beispielsweise werden aus 3-Chlorthiophen-2-carbonsäure eine Reihe von Heterocyclen synthetisiert (US-PS 4 230 873 = DE-OS 2 706 873) die u.a. als Analgetica Verwendung finden könnt. 5-Chlorthiophen-2-carbonsäure dient als Synthesekomponente zur Herstellung von Präparaten mit cholesterin- und lipidsenkender Wirkung (US-PS 4 017 514).

Als Halogenthiophen-2-carbonsäuren wurden auch schon heterocyclische Verbindungen mit Süßstoffcharakter hergestellt (US-PS 4 028 373).

Es ist bekannt, Halogenthiophen-2-carbonsäuren durch Oxidation der entsprechenden Halogen-2-acetylthiophene — auch als 1-[Halogen-(2)-thienyl]-ethanone bekannt — mittels wäßriger Permanganatlösung oder mit alkalischer, wäßriger Natriumhypochloritlösung herzustellen [J. Am. Chem. Soc., 69, 3098, (1947)]. Hierbei treten jedoch erhebliche Probleme durch den Anfall anorganischer Salze im Abwasser auf. Die Hypochloritlösung muß nämlich in 3fachem Überschuß angewendet und nach der Oxidation wieder mit Bisulfitlösung zerstört werden [Org. Synth., Coll. Vol. II, 428 (1943)]. Zur Ausfällung der freien Thiophen-2-carbonsäuren wird schließlich angesäuert.

Aus der US-PS 2 492 645 ist ein Verfahren zur Herstellung von 2-Thiophen-carbonsäure bekannt, bei dem 2-Acetyl-thiophen mittels Salpetersäure oxidiert wird. Eine maximale Ausbeute von nur bis zu 62% macht dieses Verfahren unwirtschaftlich.

Es bestand die Aufgabe, die durch Acylierung von Halogenthiophen allgemein leicht zugänglichen Halogen-2-acetylthiophene [J. Am. Chem. Soc., 69, 3093-3097 (1947)] durch eine technisch einfache Oxidationsmethode in die Halogenthiophen-2-carbonsäuren überzuführen, unter Vermeidung der genannten Probleme.

Gegenstand der Erfindung ist ein Verfahren zur Herstellung von Halogenthiophen-2-carbonsäuren der allgemeinen Formel

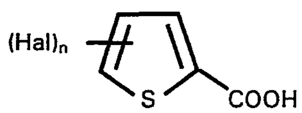

in der Hal = Halogen und n = 1 oder 2 bedeutet, durch Oxidation der entsprechenden Halogen-2-acylthiophene der allgemeinen Formel

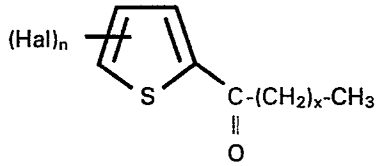

mit x = 0 bis 3, dadurch gekennzeichnet, daß die Oxidation in aliphatischen Carbonsäuren mit bis zu fünf C-Atomen, insbesondere Essigsäure, als Lösungsmittel in Gegenwart von Mn, Co, Cr oder Fe in Form von Salzen einzeln oder im Gemisch miteinander als Katalysator mittels Sauerstoff bei einer Reaktionstemperatur von 50 bis 200°C erfolgt.

Das neue Verfahren erlaubt die Herstellung von Halogenthiophen-2-carbonsäuren in hohen Ausbeuten unter Verwendung eines leicht verfügbaren Oxidationsmittels. Es besitzt weiterhin den Vorteil, daß praktisch keine umweltbelastenden Abfallsalze oder Abwässer anfallen und die Isolierung der Halogenthiophen-2-carbonsäuren auf besonders einfache Weise gelingt.

Es ist dabei überraschend, daß unter den angewandten Reaktionsbedingungen des erfindungsgemäßen Verfahrens eine Sulfonbildung nur in geringem Maße stattfindet.

Als Einsatzprodukte für die erfindungsgemäße Oxidation eignen sich sämtliche Mono- und Dihalogen-2-acylthiopene.

Als Halogenderivate werden verwendet: Fluor-, Chlor-, Brom- und Iodderivate. Aufgrund der thermischen Labilität der Iodderivate und der schweren Zugänglichkeit der Fluorderivate sind die Derivate von Chlor und Brom besonders interessant, vor allem die Chlorderivate.

Der Acylrest in den als Ausgangsmaterial verwendeten Halogen-2-acylthiophenen kann Acetyl, Propionyl, Butyryl oder Pivalyl sein. Aufgrund der leichten Verfügbarkeit von Acetanhydrid ist der Acylrest vorzugsweise Acetyl.

Als Lösungsmittel für die Durchführung der Oxidation eignen sich alle gegenüber Sauerstoff im angewendeten Temperaturbereich beständigen Lösungsmittel. Bevorzugt werden jedoch aliphatische Carbonsäuren mit bis zu fünf C-Atomen, wie z.B. Essigsäure, Propionsäure oder Pivalinsäure. Besonders bevorzugt ist Essigsäure.

Das Mengenverhältnis von Lösungsmittel zu Halogen-2-acylthiophen ist nicht kritisch und beträgt im allgemeinen ca. 4 : 1 bis 20 : 1. Ein besonders günstiger Bereich liegt bei 6 : 1 bis 12 : 1.

Als Katalysatoren werden im allgemeinen Salze von Mangan, Kobalt, Chrom oder Eisen verwendet, vor allem Halogenide, Carboxylate, Carbonate oder Sulfate dieser Metalle.

Bevorzugt sind Mangan und Kobalt, insbesondere deren Gemische, wobei sich ein Gewichtsverhältnis von Mangan : Kobalt von 10 : 1 bis 20 : 1 als günstig erweist.

Die Metallsalze werden im Lösungsmittel gelöst. Bei der bevorzugten Verwendung von aliphatischen Carbonsäuren als Lösungsmittel erweist es sich als günstig, die Carboxylate der Metalle zu verwenden, vorzugsweise jene Metallcarboxylate, die sich von der als Lösungsmittel verwendeten Carbonsäure ableiten. Besonders bevorzugte Metallsalze sind daher die Acetate.

Das Mengenverhältnis von Katalysator (gerechnet als Metall) zu Halogen-2-acylthiophen beträgt allgemein 0,4 : 100 bis 20 : 100, vorzugsweise 1 : 100 bis 10 : 100.

Als Oxidationsmittel dient Sauerstoff, entweder in

reiner Form oder im Gemisch mit Inertgasen, wie z.B. Stickstoff oder Kohlendioxid. Bevorzugt wird Luft verwendet, die mit Sauerstoff angereichert wurde, insbesondere auf etwa 50 Vol.-% Sauerstoffgehalt.

Die Reaktionstemperatur beträgt im allgemeinen 50 bis 200°C. Die optimale Reaktionstemperatur ist u.a. von der thermischen Stabilität der eingesetzten Acylderivate und der entstehenden Carbonsäurederivate abhängig und kann durch einen einfachen Vorversuch herausgefunden werden. Beim Einsatz etwa von Monohalogen-2-acetylthiophenen erweist sich ein Temperaturbereich von 80 bis 140°C als vorteilhaft.

Die Oxidation wird im allgemeinen im Druckbereich von 1 bis 20 bar durchgeführt. Es ist aber ein Vorteil des erfindungsgemäßen Verfahrens, daß im allgemeinen selbst bei druckloser Fahrweise in Reaktionszeiten bis zu ca. 6 Stunden hohe Umsätze von mindestens 90% und gute Ausbeuten von ca. 90% erreicht werden.

Die Oxidation kann sowohl diskontinuierlich als auch kontinuierlich in herkömmlichen Reaktoren wie Kesseln, Kesselkaskaden oder Blasensäulenreaktoren durchgeführt werden.

Die Aufarbeitung des Reaktionsproduktes erfolgt in einfacher Weise, indem das Lösungsmittel destillativ entfernt wird, welches nach weiterer destillativer Reinigung wieder verwendet werden kann. Das auf diese Weise als Destillationsrückstand gewonnene rohe Reaktionsprodukt wird zur Entfernung anhaftender Salze mit Wasser gewaschen. Die dabei als Festprodukte anfallenden Halogenthiophen-2-carbonsäuren können durch Umkristallisation leicht gereinigt werden.

*Beispiel 1*

Gearbeitet wird in einem ummantelten Reaktionsrohr mit Bodenfritte von 3,1 l Inhalt, einer Länge von 110 cm und einem Innendurchmesser von 60 mm. Eine Lösung von 240 g (1,5 Mol) 1-[5-Chlor-(2)-thienyl]-ethanon (Fp 48°C), 1,5 g Kobaltacetat · 4 $H_2O$ und 24,0 g Manganacetat · 4 $H_2O$ in 1,8 l Eisessig wird in das Reaktionsrohr eingefüllt und mittels Ölumlaufheizung auf 105 - 106°C erhitzt, während ein Gasstrom von 25 l Sauerstoff pro Stunde durch die Lösung geleitet wird. Die Innentemperatur steigt dann innerhalb einer Stunde auf 108 - 110°C an und wird für die weitere Versuchsdauer in diesem Bereich konstant gehalten. Ungefähr 4 Stunden nach Versuchsbeginn wird die Reaktionslösung (2116 g) heiß abgelassen und im Rotationsverdampfer bis zur Trockne eingeengt. Das trockene Rohprodukt der Carbonsäure, 220 g (90,2% Ausbeute), wird aus 10%igem wäßrigen Methanol umkristallisiert; es verbleiben 196 g an kristalliner 5-Chlorthiophen-2-carbonsäure vom Schmelzpunkt 152°C.

*Beispiel 2*

Gearbeitet wird in der gleichen Apparatur wie in Beispiel 1.

Eine Lösung von 120 g (0,75 Mol) 1-[3-Chlor-(2)-thienyl]-ethanon (Kp 85°C/2 mbar), 0,5 g Kobaltacetat · 4 $H_2O$ und 18,0 g Manganacetat · 4 $H_2O$ in 1,2 l Eisessig wird in das Reaktionsrohr eingebracht und auf eine Temperatur von 105 - 106°C erhitzt. Gleichzeitig wird ein Gasstrom von 25 l Sauerstoff pro Stunde durch die Lösung geleitet, so daß innerhalb einer Stunde die Innentemperatur auf 108 bis 110°C ansteigt und dort für die weitere Versuchsdauer konstant gehalten wird. Etwa 3,5 Stunden nach Versuchsbeginn wird die Reaktionslösung (1322 g) heiß abgelassen und wie in Beispiel 1 aufgearbeitet. Die verbleibenden 88 g Rohprodukt werden aus 3%iger Essigsäure umkristallisiert. Als Kristallisat verbleiben 68 g 3-Chlorthiophen-2-carbonsäure, entsprechend einer Ausbeute von 55,8%, bezogen auf eingesetztes Ausgangsprodukt. Da das eingesetzte Keton-Gemisch jedoch nur etwa 80 Gew.-% 1-[3-Chlor-(2)-thienyl]-ethanon enthält, errechnet sich für die erhaltene 3-Chlorthiophen-2-carbonsäure eine Selektivität von 70%. Aus Wasser umkristallisiert, schmilzt das Produkt bei 188 - 190°C.

*Beispiel 3*

Man arbeitet wie in Beispiel 1, aber mit dem Unterschied, daß statt reinem Sauerstoff ein Gasgemisch von15 l Sauerstoff und 15 l Stickstoff pro Stunde durch die Lösung geleitet wird. Nach einer Versuchsdauer von etwa 4,5 Stunden wird die heiße Reaktionslösung abgelassen und wie in Beispiel 1 aufgearbeitet. Man erhält schließlich 228 g trockenes Rohprodukt und nach Umkristallisation aus 15%igem Methanol 205 g kristalline 5-Chlorthiophen-2-carbonsäure, entsprechend einer Reinausbeute von 84,0%.

**Patentansprüche**

1. Verfahren zur Herstellung von Halogenthiophen-2-carbonsäuren der allgemeinen Formel

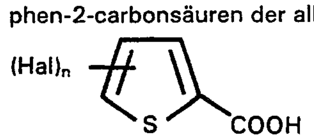

in der Hal = Halogen und n = 1 oder 2 bedeutet, durch Oxidation der entsprechenden Halogen-2-acylthiophene der allgemeinen Formel

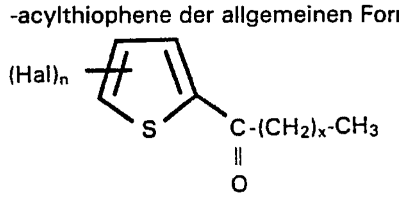

mit x = 0 bis 3, dadurch gekennzeichnet, daß die Oxidation in aliphatischen Carbonsäuren mit bis zu fünf C-Atomen, insbesondere Essigsäure, als Lösungsmittel in Gegenwart von Mn, Cro, Cr oder Fe in Form von Salzen einzeln oder im Gemisch miteinander als Katalysator mittels Sauerstoff bei einer Reaktionstemperatur von 50 bis 200°C erfolgt.

2. Verfahren nach Anspruch 1, dadurch gekennzeichnet, daß das Mengenverhältnis des Katalysators (gerechnet als Metall) zu Halogen-2-acylthiophen 0,4 : 20 bis 20 : 100 beträgt.

3. Verfahren nach einem der Ansprüche 1 oder 2, dadurch gekennzeichnet, daß als Oxidationsmittel mit Sauerstoff angereicherte Luft eingesetzt wird.

4. Verfahren nach einem der Ansprüche 1 bis 3, dadurch gekennzeichnet, daß als Ausgangsverbindungen die Halogen-2-acetylthiophene (x = 0) eingesetzt werden.

5. Verfahren nach einem der Ansprüche 1 bis 4, dadurch gekennzeichnet, daß ein Gemisch von Mangan- und Kobaltsalz als Katalysator verwendet wird.

6. Verfahren nach Anspruch 5, dadurch gekennzeichnet, daß das Gewichtsverhältnis Mangan : Kobalt = 10 : 1 bis 20 : 1 ist.

## Claims

1. A process for the preparation of halothiophene-2-carboxylic acids of the general formula

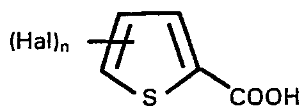

in which Hal denotes halogen and n denotes 1 or 2, by oxidation of the appropriate halo-2-acylthiophenes of the general formula

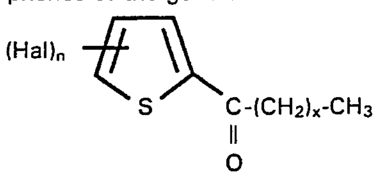

where x = 0 to 3, which comprises carrying out the oxidation in an aliphatic carboxylic acid having up to five carbon atoms, in particular acetic acid as the solvent, in the presence of Mn, Co, Cr or Fe in the form of salts, either alone or mixed with each other, as the catalyst, using oxygen at a reaction temperature of 50 to 200°C.

2. The process as claimed in claim 1, wherein the amount ratio of catalyst (calculated as the metal) to halo-2-acylthiophene is 0.4 : 20 to 20 : 100.

3. The process as claimed in either of claims 1 or 2, wherein oxygen-enriched air is employed as oxidant.

4. The process as claimed in any one of claims 1 to 3, wherein the halo-2-acetylthiophenes (x = 0) are employed as starting compounds.

5. The process as claimed in any one of claims 1 to 4, wherein a mixture of manganese and cobalt salt is used as catalyst.

6. The process as claimed in claim 5, wherein the weight ratio of manganese : cobalt is 10 : 1 to 20 : 1.

## Revendications

1. Procédé pour préparer des acides halogénothiophène-carboxyliques-2 répondant à la formula générale:

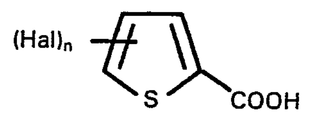

dans laquelle Hal représente un halogène et n est égal à 1 ou à 2, par oxydation des acyl-2 halogène-thiophènes correspondants de formule générale:

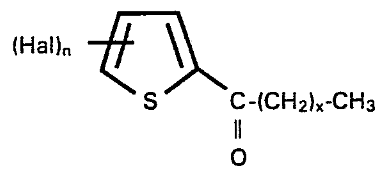

dans lesquels x désigne un nombre de 0 à 3, procédé caractérisé en ce qu'on effectue l'oxydation au moyen d'oxygène, à une température réactionnelle de 50 à 200°C, en utilisant, comme solvants, des acides carboxyliques aliphatiques contenant au maximum 5 atomes de carbone, plus particulièrement l'acide acétique, et, comme catalyseur, Mn, Co, Cr ou Fe sous la forme de sels, isolément ou en mélange entre eux.

2. Procédé selon la revendication 1, caractérisé en ce que le rapport entre la quantité du catalyseur (considéré à l'état de métal) et la quantité de l'acyl-2 halogène-thiophène est compris entre 0,4 : 20 et 20 : 100.

3. Procédé selon l'une des revendications 1 et 2, caractérisé en ce qu'on utilise, comme agent d'oxydation, de l'air enrichi en oxygène.

4. Procédé selon l'une quelconque des revendications 1 à 3, caractérisé en ce qu'on utilise comme corps de départ les acétyl-2 halogéno-thiophènes (x = 0).

5. Procédé selon l'une quelconque des revendications 1 à 4, caractérisé en ce qu'on utilise, comme catalyseur, un mélange d'un sel de manganèse et d'un sel de cobalt.

6. Procédé selon la revendication 5, caractérisé en ce que le rapport pondéral entre le manganèse et le cobalt est compris entre 10 : 1 et 20 : 1.